# EUROPEAN PATENT APPLICATION

(11) **EP 4 741 410 A1**
(43) Date of publication of application: **13.05.2026**
(21) Application number: 24836103.2
(22) Date of filing: 04.07.2024
(51) Int. Cl.: C07H 5/06, C07K 14/54, C07K 14/705, C08B 37/08

(54) **SOLUBILIZATION GROUP FOR POORLY WATER-SOLUBLE PROTEINS**

(30) Priority: 06.07.2023 JP 2023111400
(71) Applicant: Glytech, Inc., Kyoto-shi, Kyoto 600-8813 (JP)
(72) Inventor: KAJIHARA, Yasuhiro, Toyonaka-shi, Osaka 560-0043 (JP); OKAMOTO, Ryo, Toyonaka-shi, Osaka 560-0043 (JP); MAKI, Yuta, Toyonaka-shi, Osaka 560-0043 (JP); LIU, Yanbo, Toyonaka-shi, Osaka 560-0043 (JP); FUJITA, Shoichi, Kyoto-shi, Kyoto 600-8813 (JP); ELOUALI, Sofia, Kyoto-shi, Kyoto 600-8813 (JP)
(74) Representative: Greaves Brewster LLP
(86) International application number: PCT/JP2024/024239
(87) International publication number: WO 2025/009591

(57) **Abstract**

The object of the present invention is to provide novel means that can be used for solubilizing proteins with poor water solubility. Provided is a hydrophilic solubilizing group for proteins or peptides, comprising a sugar chain moiety; and a functional group moiety that is bound to the amino or carboxy group of said sugar chain, and that binds to the amino acid side chain and can be detached from said protein or amino acid side chain in the presence of an acid.

## Description

### Technical Field

The present disclosure relates to a solubilizing group for solubilizing proteins with poor water solubility, as well as a method for producing solubilized proteins etc. employing the said solubilizing group.

### Background Art

There has conventionally been great need for solubilization of proteins. For example, when a protein is to be used as a pharmaceutical active ingredient, it is preferred that the protein is sufficiently soluble in water, in terms of e.g. improvement in absorbability by systemic administration (such as oral administration), enrichment of the active ingredient in formulation, and realization of appropriate viscosity in formulation. Moreover, even at the drug discovery stage, solubilization of proteins is again necessary for protein folding or analysis of complexes of folded proteins and disease-causing target molecules. On the other hand, due to advancement and sophistication of recent drug discovery technology, candidate molecules with poor water solubility are increasingly growing, and the need for solubilization technology is further being escalated.

As an approach for solubilizing proteins with poor water solubility, an approach employing a hydrophilic tag is known. For example, Patent Literature 1 describes a hydrophilic polylysine tag for solubilizing proteins with poor water solubility.

### Citation List

### Non-Patent Literature

[Non-Patent Literature 1] Tsuda et al., Angew. Chem. Int. Ed. 2018, 57, 2105-2109

### Summary of the Invention

### Problems to be Solved by the Invention

The object of the present disclosure is to provide novel means that can be used for solubilizing proteins with poor water solubility.

### Means for Solving the Problems

Sugar chains are known to bind to membrane proteins or secretory proteins etc. *in vivo,* to have various influences on their biological activity. On the other hand, sugar chains, in contrast to their usefulness, were restricted in their use due to difficulty in acquisition and high cost.

The inventors of the present application, in the process of repeated extensive investigation in order to solve the above problem, found that proteins with poor water solubility can be solubilized by adding a sugar chain solubilizing group to proteins with poor water solubility. Further, in doing so, it was found that by retaining a particular functional group moiety on the sugar chain solubilizing group, the sugar chain solubilizing group employed for solubilization can be separated/collected and re-utilized after obtaining the solubilized protein of interest, thus coming to complete the invention.

That is, the present disclosure encompasses the following characteristics:
(1) A hydrophilic solubilizing group for proteins or peptides, comprising a sugar chain moiety; and a functional group moiety that is bound to the amino or carboxy group of said sugar chain, and that binds to the amino acid side chain and can be detached from said protein or amino acid side chain in the presence of an acid.
(2) The solubilizing group according to (1), comprising a sugar chain moiety; and a triphenylmethanol moiety bound to the amino or carboxy group of said sugar chain.
(3) The solubilizing group according to (2), wherein said triphenylmethanol moiety is bound to the carboxy group of said sugar chain.
(4) The solubilizing group according to (1), wherein said sugar chain is characterized in that it consists of 4 or more sugars.
(5) The solubilizing group according to (1), wherein said sugar chain is characterized in that it consists of 7 or more sugars.
(6) The solubilizing group according to (1), wherein said sugar chain is characterized in that it consists of 9 or more sugars.
(7) The solubilizing group according to (1), wherein said sugar chain is characterized in that it is a complex type bianntennary sugar chain, a complex type triantennary sugar chain, or a complex type tetraantennary sugar chain.
(8) The solubilizing group according to (1), wherein said sugar chain is characterized in that it is a complex type bianntennary sugar chain.
(9) The solubilizing group according to (8), wherein said complex type bianntennary sugar chain is characterized in that it is a sugar chain selected from the group consisting of a disialo sugar chain, a monosialo sugar chain, an asialo sugar chain, a diGlcNAc sugar chain, and a dimannose sugar chain.
(10) The solubilizing group according to (1), wherein said sugar chain is characterized in that it is a sugar chain represented by the following formula: 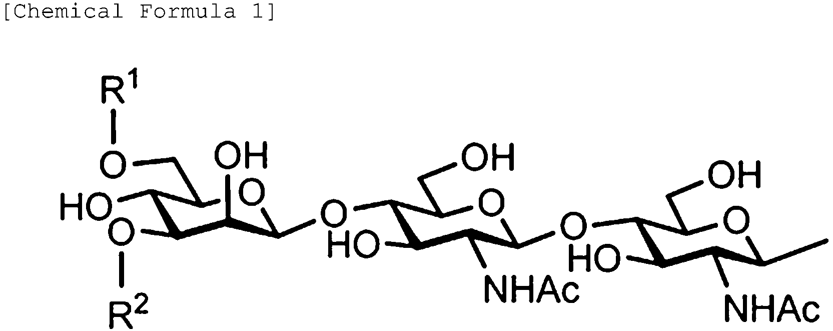 [wherein R¹ and R² are the same or different, to indicate 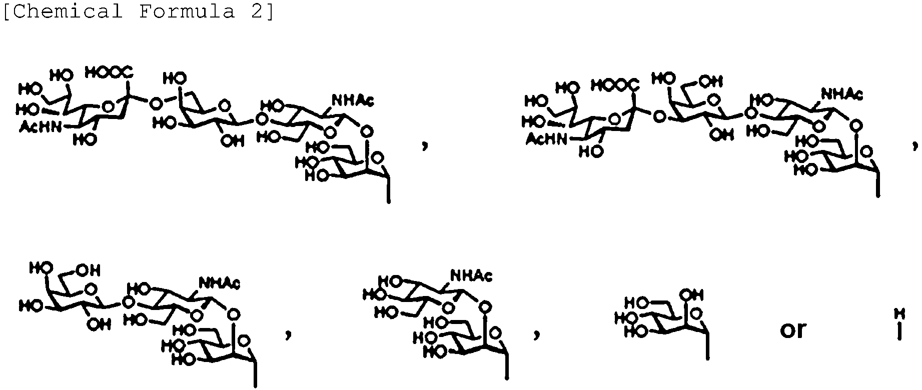 , and Ac indicates an acetyl group].
(11) The solubilizing group according to (1), having the following structure. 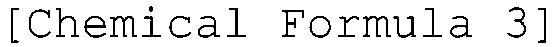 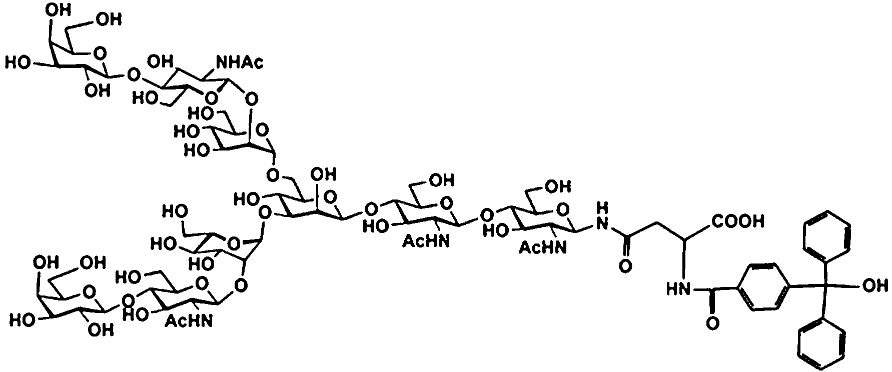
(12) A peptide or protein having one or more of the solubilizing groups according to (1).
(13) The peptide or protein according to (12), which has poor water solubility.
(14) The peptide or protein according to (12), selected from the group consisting of interleukin and transmembrane protein, and as a fragment thereof.
(15) The peptide or protein according to (14), which is rhodopsin or IL-6, or a fragment thereof.
(16) A method for producing a solubilized protein or peptide, comprising a step of binding the solubilizing group according to (1) to the sulfhydryl group, the carboxy group, or the amino group of one or more amino acid side chains constituting any protein or peptide.
(17) A method for producing the protein or peptide of interest employing proteins or peptides with poor water solubility, comprising:
   a step of binding the solubilizing group according to (1) to the sulfhydryl group, the carboxy group, or the amino group of one or more amino acid side chains constituting said proteins or peptides with poor water solubility to allow solubilization, and
   a step of linking said solubilized protein or peptide with another soluble protein or peptide, or solubilized protein or peptide, to obtain the protein of interest.
(18) The method according to (17), further comprising:
   a step of detaching said solubilizing group from said protein of interest in the presence of an acid, and
   a step of collecting said solubilizing group.

### Effects of the Invention

According to the present disclosure, novel means that can be used for solubilizing proteins etc. with poor water solubility is provided.

### Brief Description of the Drawings

Figure 1 shows the result by HPLC purification of the produced sugar chain solubilizing group 1.
Figure 2 shows the reaction progress of sugar chain solubilizing group 1 and peptide 2, as well as the result by HPLC purification of the reactant thereof, compound 3.
Figure 3 shows the deprotection reaction progress of the sugar chain solubilizing group 1 from compound 3, as well as the result by HPLC purification of the post-reaction product thereof, peptide 2.
Figure 4 shows the SUMO tag protein removal reaction progress from compound 3, as well as the result by HPLC purification of the post-reaction product thereof compound 4, and SUMO tag protein.
Figure 5 shows the reaction progress of sugar chain solubilizing group 1 and IL-6 N-terminal peptide, as well as the result by HPLC purification of the post-reaction product thereof, compound 6.
Figure 6 shows the deprotection reaction progress of the sugar chain solubilizing group 1 from compound 6, as well as the result by HPLC purification of the post-reaction product thereof compound 5, and sugar chain solubilizing group 1.
Figure 7 shows the peptide linking reaction progress of peptide 6 having sugar chain solubilizing group 1 and the solubilized peptide 7, as well as the result by HPLC purification of the post-reaction product thereof, compound 8.
Figure 8 shows the desulfurization reaction progress of compound 8, as well as the result by HPLC purification of the post-reaction product thereof, compound 9.
Figure 9 shows the HPLC chart of the post-reaction product compound 10 of the deprotection reaction of the sugar chain solubilizing group 1 from compound 9.
Figure 10 shows the reaction progress of the folding reaction of compound 10, as well as the HPLC chart of the post-reaction product thereof, compound 11.

### Description of Embodiments

The contents of the present disclosure will now be described in detail below.

The present disclosure relates to a hydrophilic solubilizing group for solubilizing proteins (also referred to herein as simply the "solubilizing group according to the present disclosure").

In the present disclosure, a "protein" may comprise a protein or a portion thereof (i.e. peptide), as well as derivatives thereof. A protein is typically a protein with poor water solubility. A derivative of a protein refers to a derivative from a natural protein, and includes, but is not limited to, e.g., those where a portion of amino acids constituting a natural protein is replaced with a non-natural amino acid, those having a chemical or genetic treatment is applied to a natural protein, a combination of natural protein fragments, and the like.

In the present disclosure, proteins include any protein with poor water solubility that needs to be solubilized, and may non-limitingly include interleukins such as IL-1, IL-2, and IL-6, various chemokines, interferons, other cytokines such as lymphokines, transmembrane proteins such as rhodopsin, as well as fragments thereof.

The solubilizing group according to the present disclosure is characterized in that it comprises a sugar chain moiety; and a functional group moiety that is bound to the amino or carboxy group of said sugar chain, and that binds to the amino acid side chain and can be detached from said protein or amino acid side chain in the presence of an acid.

In the present disclosure, the functional group can include, but not limited to, e.g., triphenylmethanol, methoxybenzyl, phenacyl, and diphenylmethyl. Said functional group moiety also further has a carboxy group, an amino group, a hydroxyl group, and the like for binding to the sugar chain moiety.

In the present disclosure, "in the presence of an acid" refers to the acidic condition generally employed in the deprotection of a protecting group. Acids that can be used in the present disclosure can include, but are not limited to, e.g., inorganic acids such as hydrochloric acid and sulfuric acid, Lewis acids such as boron trifluoride diethylether (BF3·OEt2), dimethyl (methylthio)sulfonium trifluoromethanesulfonate (DMTST), trimethylsilyl trifluoromethanesulfonate, triethylsilyl trifluoromethanesulfonate, tripropylsilyl trifluoromethanesulfonate, dimethylethylsilyl trifluoromethanesulfonate, tribenzylsilyl trifluoromethanesulfonate, trinaphthylsilyl trifluoromethanesulfonate, or tribenzylmethylsilyl trifluoromethanesulfonate, silver trifluoromethanesulfonate, cyclopentadienyl hafnium chloride, cyclopentadienyl zirconium chloride, and tin chloride, organic acids such as formic acid, acetic acid, trifluoroacetic acid, trifluoroacetic anhydride, trifluoromethanesulfonic acid, and tetrafluoromethanesulfonic acid, and the like. These acids can be used in a combination of one or two or more types. Moreover, those skilled in the art can appropriately adjust the amount of acids used, and it is preferred to be employed in excess amounts relative to the solubilized protein.

In one aspect of the present disclosure, the solubilizing group comprises a sugar chain moiety; and a triphenylmethanol moiety bound to the amino or carboxy group of said sugar chain. In a more specific embodiment of the present disclosure, the solubilizing group comprises a triphenylmethanol moiety bound to the carboxy group of said sugar chain.

In the present disclosure, "sugar chain" refers to a compound composed of a stretch of one or more unit sugars (monosaccharides and/or derivatives thereof). When there is a stretch of two or more unit sugars, each of the unit sugars are bound by dehydration condensation via glycoside bond. Such sugar chains include, but are not limited to, a wide range, such as monosaccharides and polysaccharides contained *in vivo* (glucose, galactose, mannose, fucose, xylose, N-acetylglucosamine (GlcNAc), N-acetylgalactosamine (GalNAc), sialic acid, and complexes and derivatives thereof), as well as degraded polysaccharides, glycoproteins, proteoglycans, glycosaminoglycans, sugar chains degraded or induced from complex biomolecules such as glycolipids, and the like. Sugar chains may be linear or branched.

Moreover, in the present disclosure, "sugar chains" also include sugar chain derivatives, and sugar chain derivatives include, but are not limited to, sugar chains that are e.g., sugar in which the sugar constituting the sugar chain has a carboxy group (such as aldonic acid wherein the C-1 position is oxidized to become carboxylic acid (such as D-glucose oxidized to D-gluconic acid) and uronic acid wherein the terminal C atom becomes a carboxylic acid (D-glucose oxidized to D-glucuronic acid)), sugar having an amino group or an amino group derivative (such as acetylated amino group) (such as N-acetyl-D-glucosamine, N-acetyl-D-galactosamine, and the like), sugar having both amino and carboxy groups (such as N-acetylneuramic acid (sialic acid), N-acetylmuramic acid, and the like), deoxylated sugar (such as 2-deoxy-D-ribose), sulfated sugar comprising a sulfate group, phosphorylated sugar comprising a phosphate group, and the like.

In the present disclosure, the type of the unit sugar constituting the sugar chain is not particularly limited, as long as it can confer sufficient solubility to the protein with poor water solubility depending on the solubility thereof. In one embodiment, the sugar chain according to the present disclosure comprises at least two mannoses.

The sugar chain in the present disclosure may also be a sugar chain that exists *in vivo* as a glycoconjugate (glycopeptide (or glycoprotein), proteoglycan, glycolipid, and the like), or it may be a sugar chain that does not exist *in vivo* as a glycoconjugate.

A sugar chain that exists *in vivo* as a glycoconjugate is preferred when it is employed for e.g. adding the solubilizing group according to the present disclosure to a protein with poor water solubility, and solubilizing it for *in vivo* administration. Such sugar chains include N-linked sugar chains, O-linked sugar chains, and the like, which are sugar chains that are bound *in vivo* as glycopeptides (or glycoproteins) to peptides (or proteins), and preferably, N-linked sugar chains are employed. N-linked sugar chains can include, e.g., high-mannose, complex, and hybrid types, and it is preferably a complex type.

In one embodiment, the complex sugar chain used in the present disclosure includes, e.g., a sugar chain represented by the following general formula: 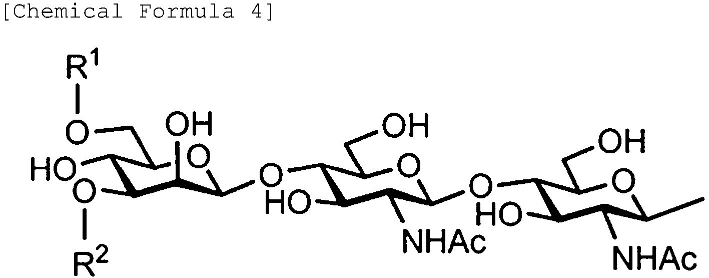 [wherein R¹ and R² are the same or different, to indicate 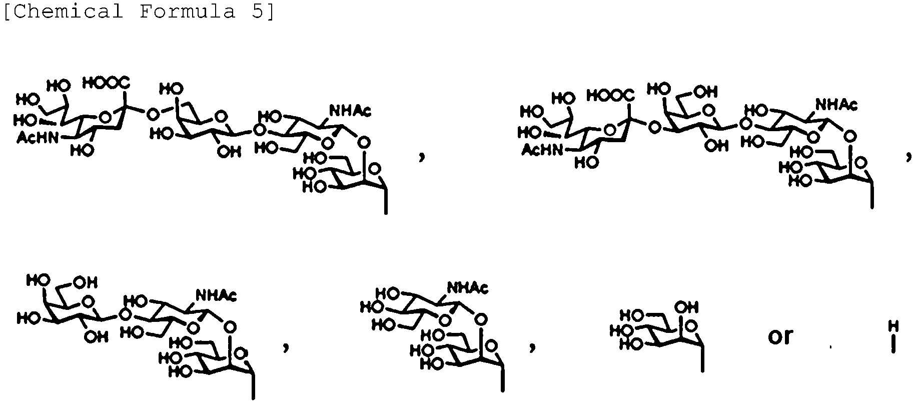 . Ac indicates an acetyl group].

In one aspect of the present disclosure, the number of sugar chains is not restricted as long as it comprises sugars that are sufficient to render sufficient solubility to the protein with poor water solubility, and it is preferred that it is a sugar chain consisting of 4 or more, such as 5 or more, 7 or more, 9 or more, or 11 or more sugars.

In another embodiment of the present disclosure, the sugar chain is a sugar chain consisting of 4 - 11, 7 - 11, such as 9 sugars.

In one aspect of the present disclosure, the sugar chain is a complex type bianntennary sugar chain. A complex sugar chain comprises two or more types of monosaccharides, and is characterized in having the basic structure indicated below and a lactosamine structure indicated by Galβ1-4GlcNAc.

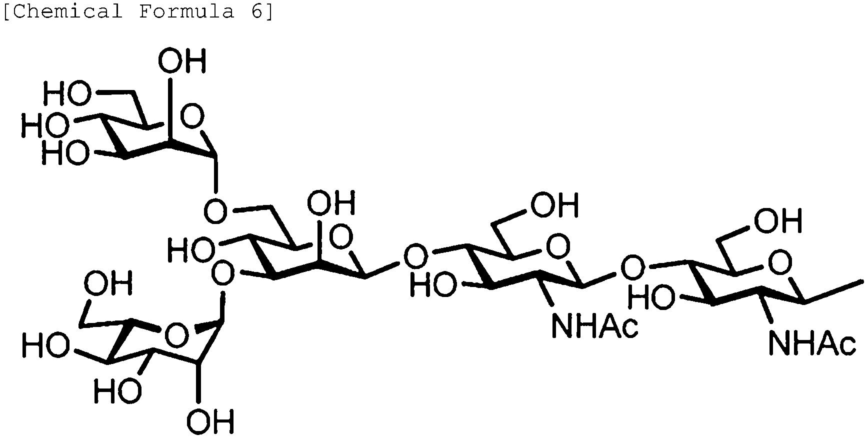

A complex type bianntennary sugar chain refers to those having single-stranded sugar chains each of which consisting of 0 - 3 sugars bound to the two mannoses at the terminal of the basic structure. Complex type bianntennary sugar chain can include, e.g., a disialo sugar chain indicated below 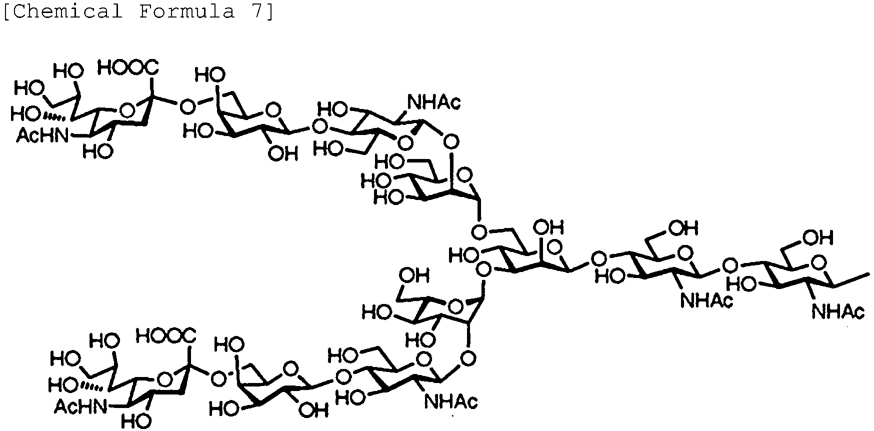 , monosialo sugar chains 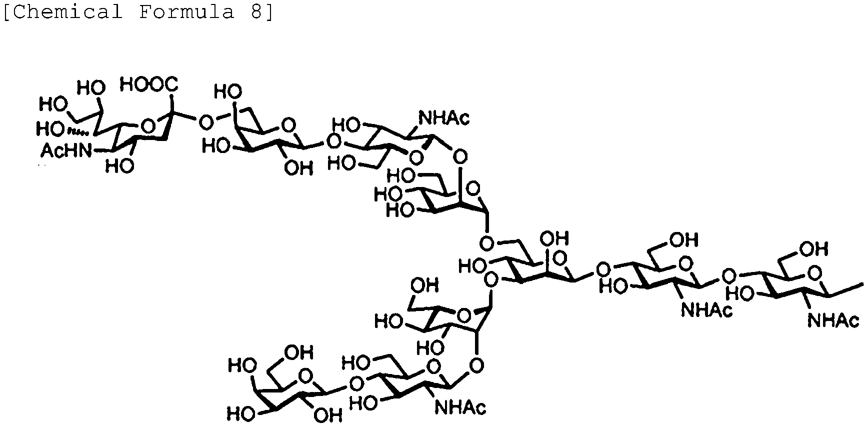 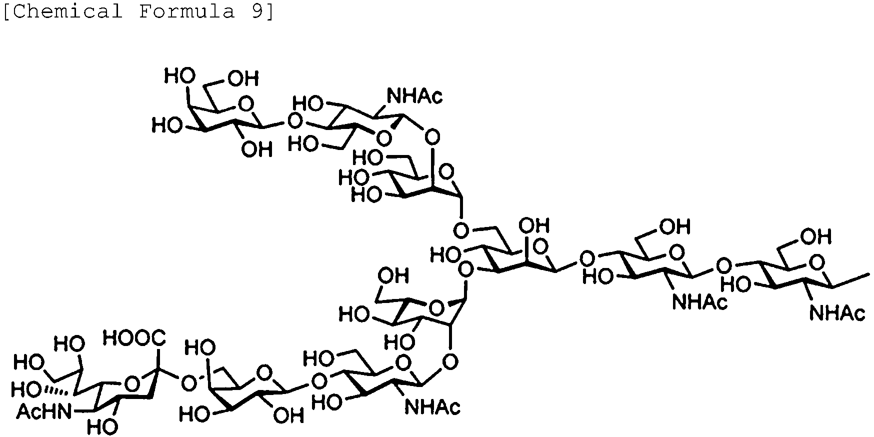 , an asialo sugar chain 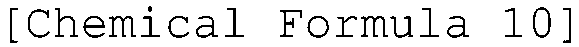 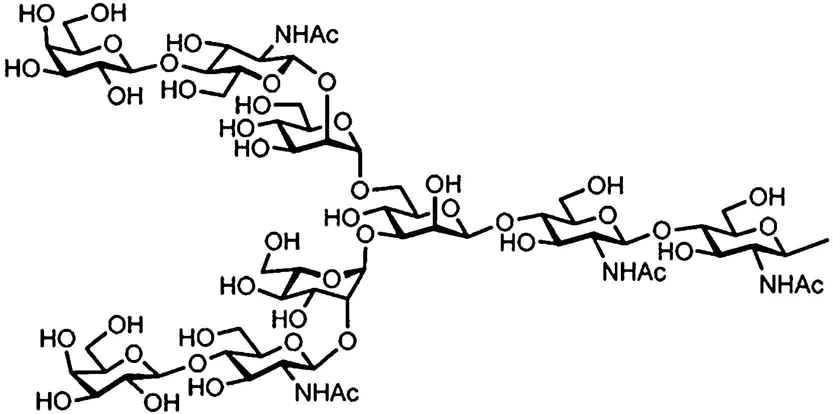 , a diGlcNAc sugar chain 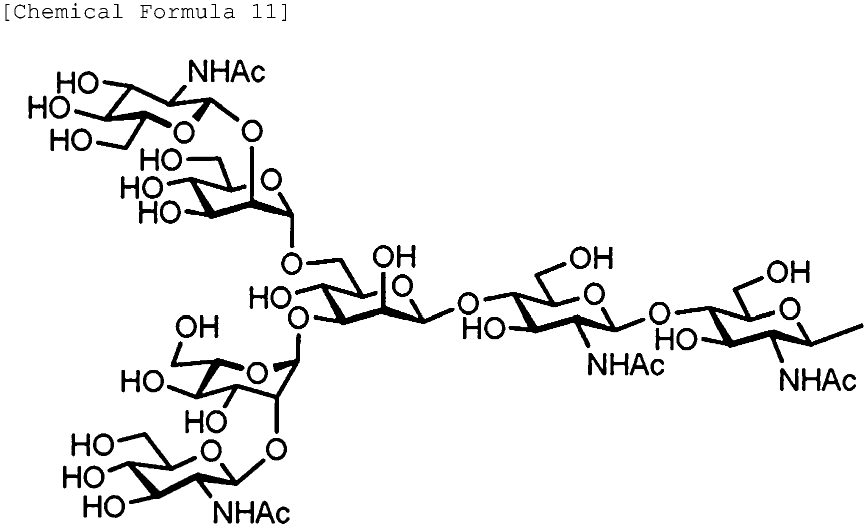 , a dimannose sugar chain 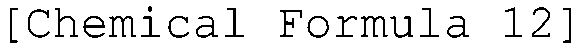 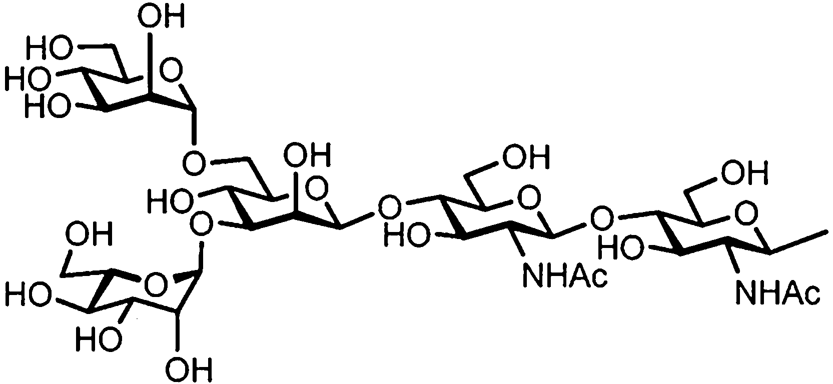 , and the like. In one embodiment of the present disclosure, the complex type bianntennary sugar chain is an asialo sugar chain.

Moreover, the complex type sugar chain of the present disclosure includes complex type bianntennary sugar chains, as well as complex type triantennary sugar chains, and complex type tetraantennary sugar chains). For example, complex type triantennary sugar chains and complex type tetraantennary sugar chains can include trisialo sugar chains represented by the following structural formulae, and a tetrasialo sugar chain represented by the following structural formula: 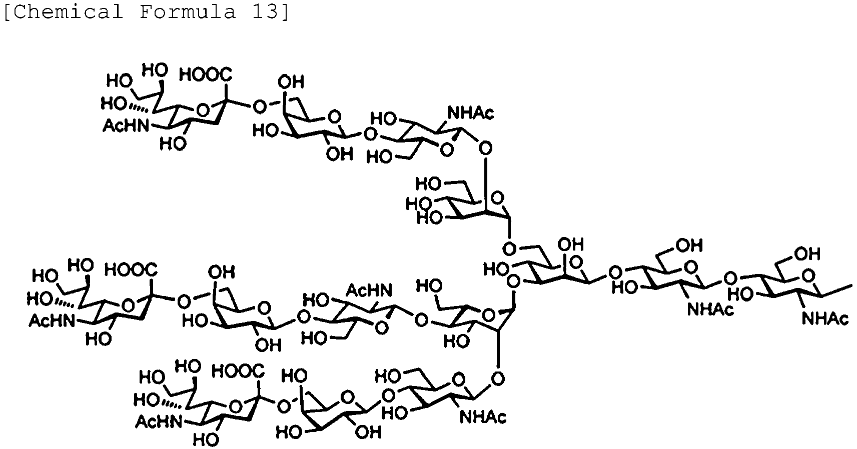 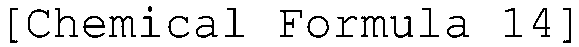 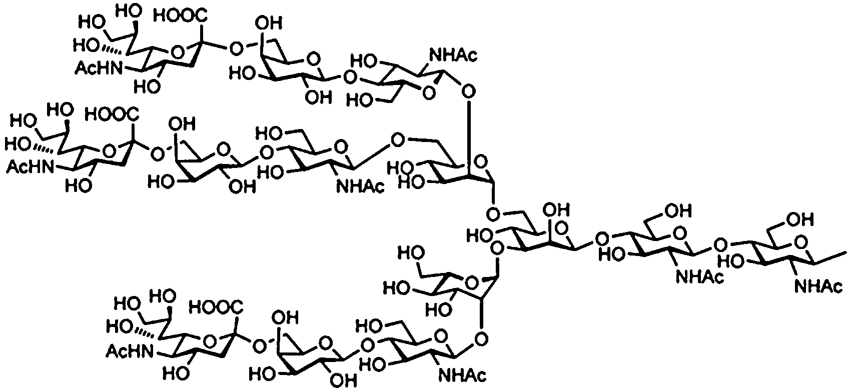 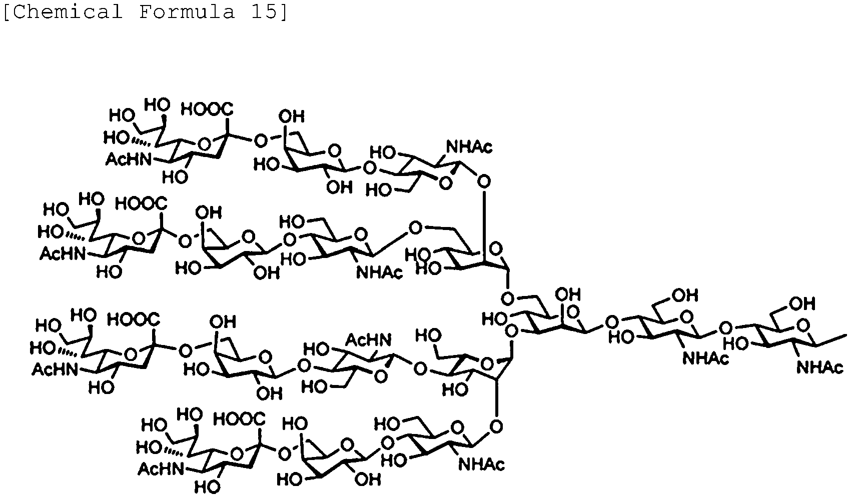 Moreover, complex type triantennary sugar chains and complex type tetraantennary sugar chains can also include sugar chains having lost one or more sugars from the non-reducing terminal of these trisialo sugar chains or tetrasialo sugar chains.

Further, the complex sugar chain of the present disclosure includes those having a fucose added. Complex sugar chains having a fucose added can include fucose-containing complex sugar chains represented by the following structural formulae 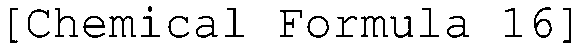 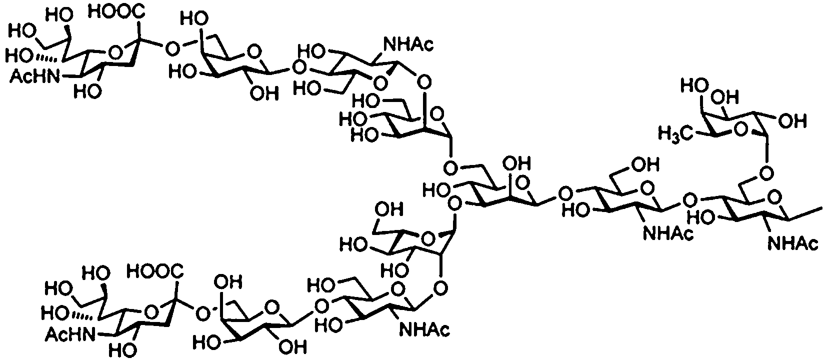 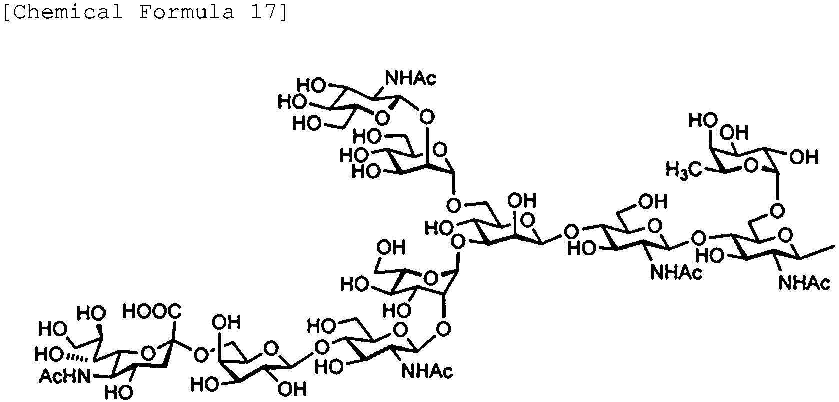 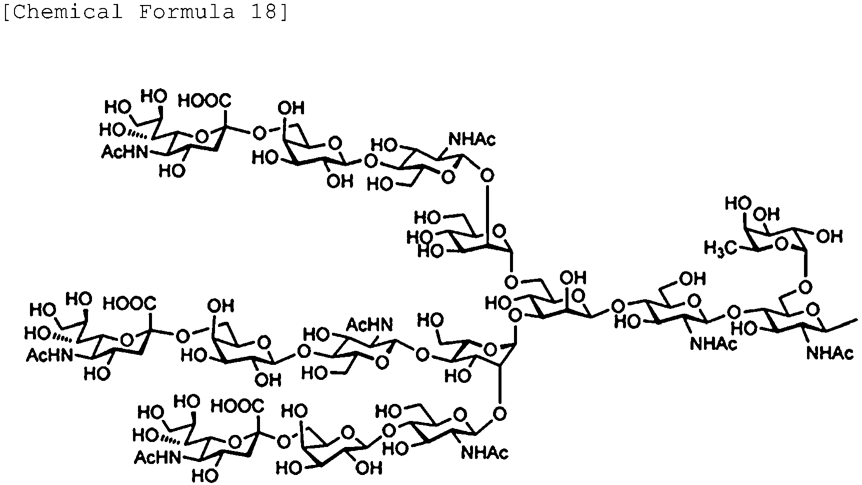 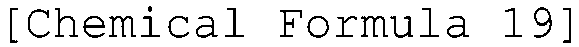 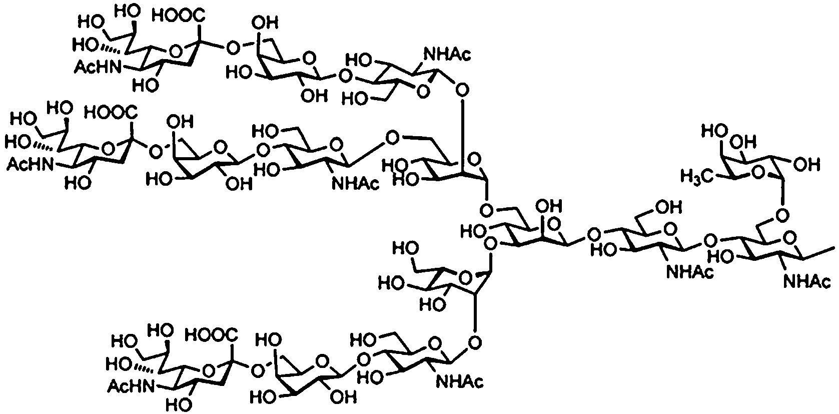 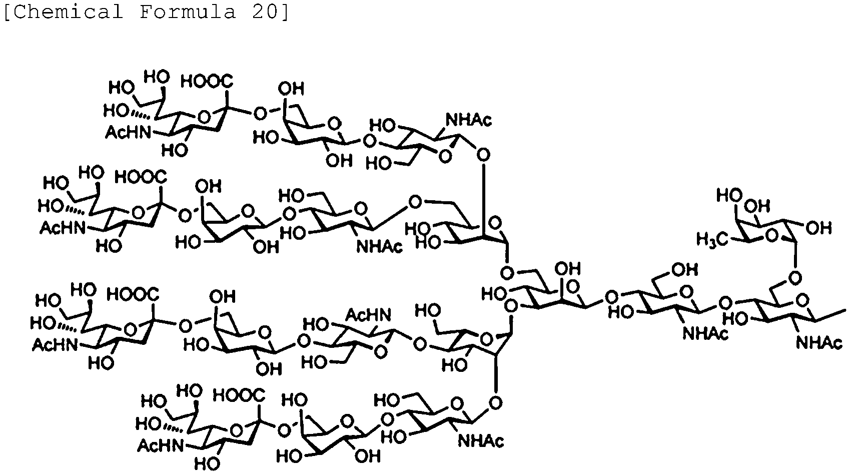 . Moreover, sugar chains having lost one or more sugars from the non-reducing terminal of these fucose-containing complex sugar chains can also be included.

Moreover, in the present disclosure, "disialo sugar chain", "monosialo sugar chain", "asialo sugar chain", "diGlcNAc sugar chain", "dimannose sugar chain", "complex type bianntennary sugar chain", "complex type triantennary sugar chain", "complex type tetraantennary sugar chain", and "fucose-containing complex sugar chain" also include, in addition to those indicated by the above chemical formulae, those having a different binding mode to the examples indicated by the chemical formulae, and such sugar chains are also included in the sugar chain of the present disclosure. Such sugar chains include, e.g., disialo sugar chain or monosialo sugar chain in which sialic acid and galactose are bound by (α2 -> 3) bond, and the like.

Moreover, in the case of a sugar chain having sialic acid at the non-reducing terminal of the sugar chain, a sugar chain having the carboxy group of the sialic acid modified can also be employed. Modification of the carboxy group of the sialic acid is preferably a group that can abolish the negative charge of the carboxy group, or convert it into a positive charge, and can include, e.g., an alkylamino group having 1 - 30 carbons, a benzyl group, an amino group, an aminoethylamino group, and the like. By introducing these modifying groups to abolish the negative charge of the carboxy group of the sialic acid (such as benzyl group and amino group), or to convert it into a positive charge (such as aminoethylamino group), controlling of blood clearance or body distribution of glycosylated polypeptides can be contemplated.

Moreover, high-mannose sugar chains employed in the present disclosure are sugar chains further having two or more mannoses bound to the basic structure of complex sugar chain described above. Since high-mannose sugar chains are bulky, stability in blood may be further increased by binding a high-mannose sugar chain to a peptide. Sugar chains comprising 5 - 9 mannoses as with mammalian high-mannose sugar chains are preferred, although it may also be sugar chains comprising more mannoses as with yeast high-mannose sugar chains. High-mannose sugar chains preferably employed in the present disclosure can include, e.g., high-mannose-5 (M-5) 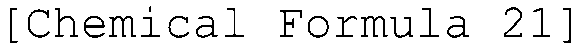 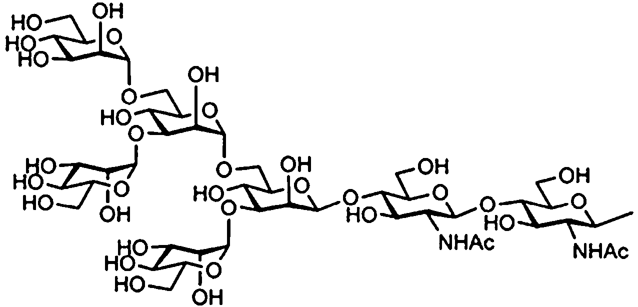 , high-mannose-9 (M-9) 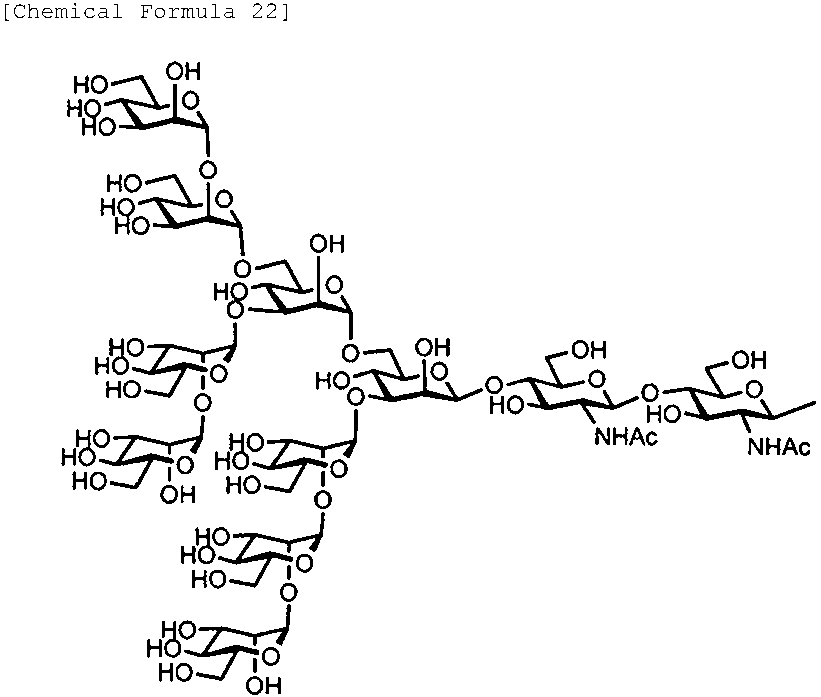 , and the like.

In the present disclosure, preferred sugar chains can include, e.g., sugar chains indicated below, which are sugar chains having identical structure (sugar chains having identical constituent sugar type and binding mode thereof) to, or sugar chains having lost one or more sugars from the non-reducing terminal of, sugar chains that exist as glycoproteins bound to proteins in the human body (such as the sugar chain described in "FEBS LETTERS Vol. 50, No. 3, Feb. 1975").

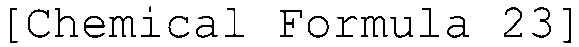

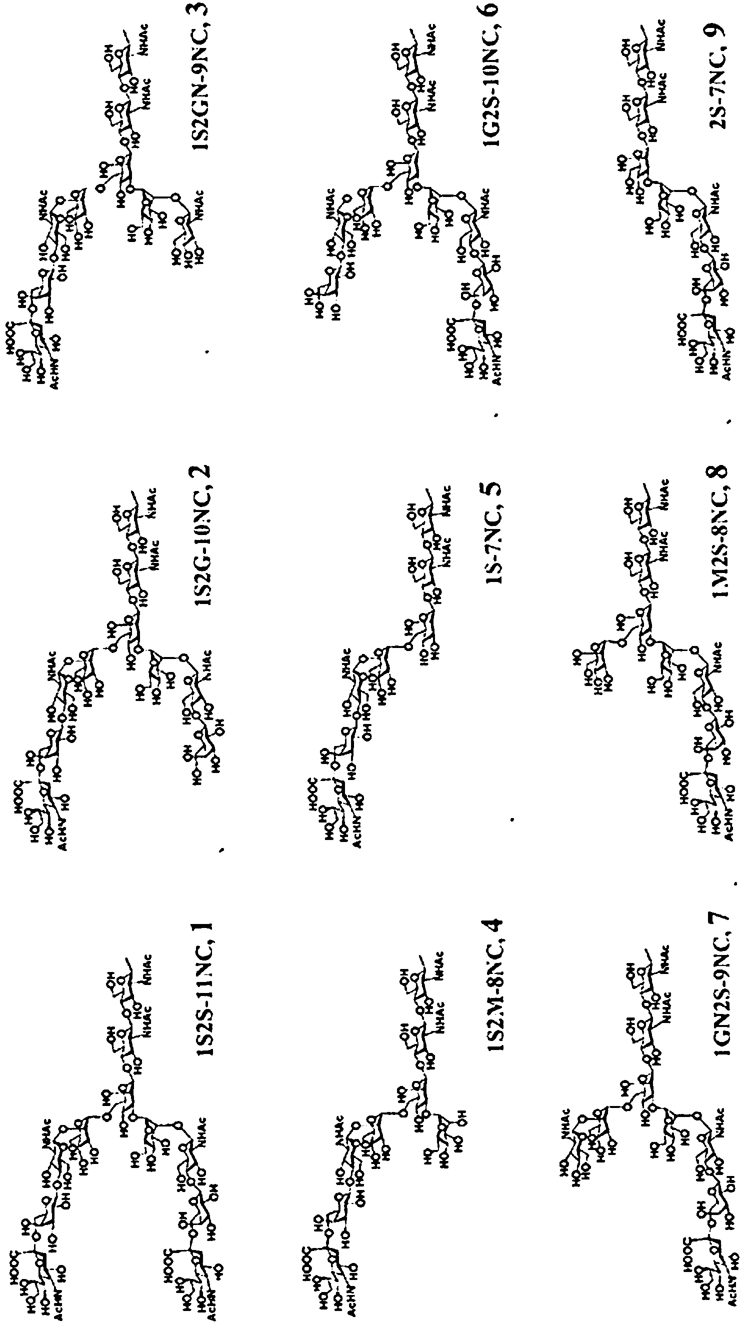

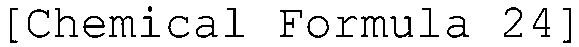

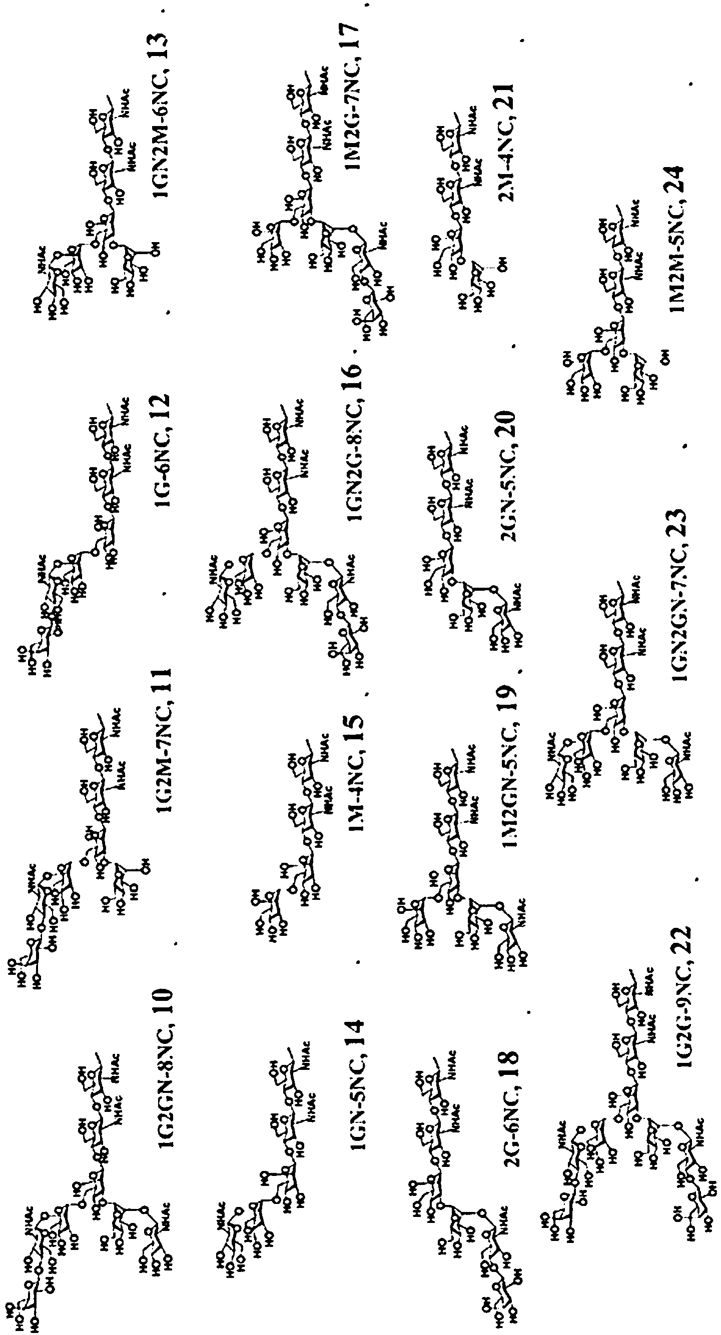

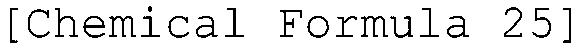

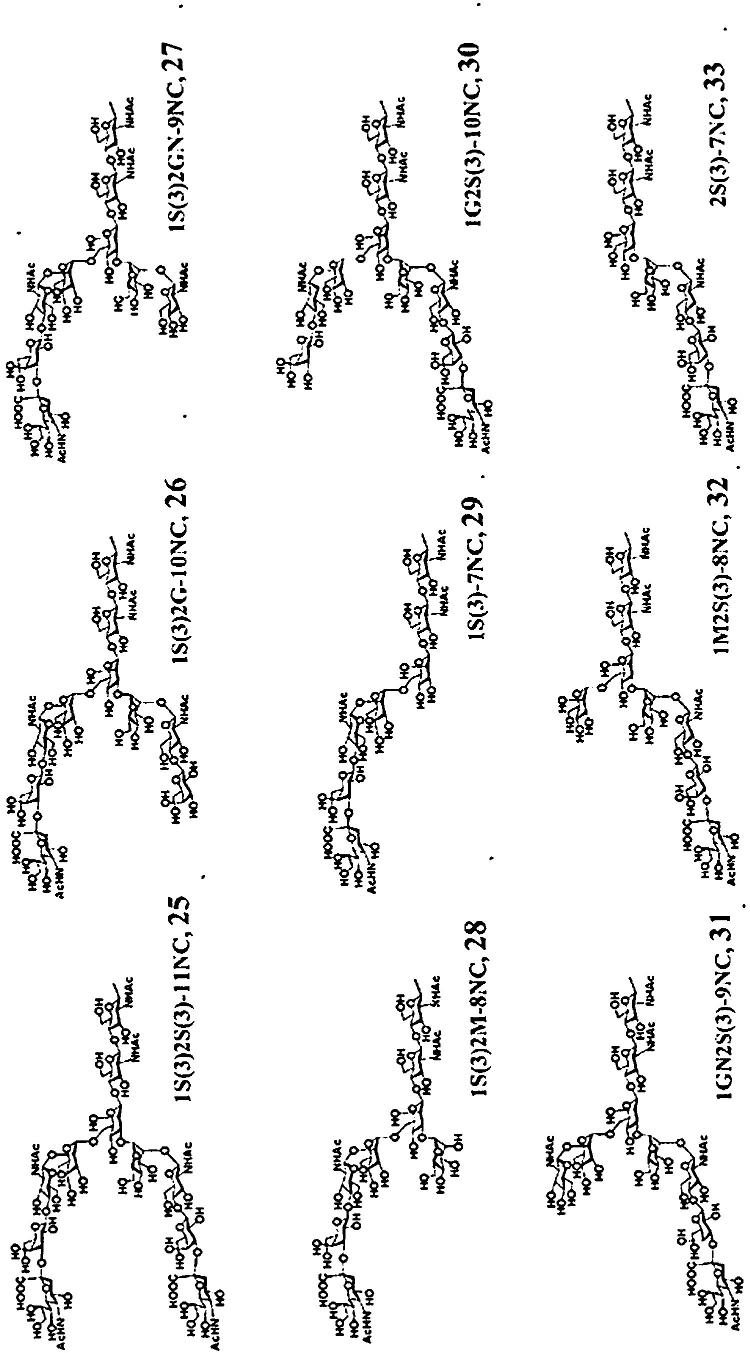

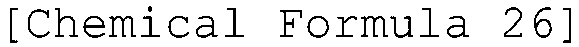

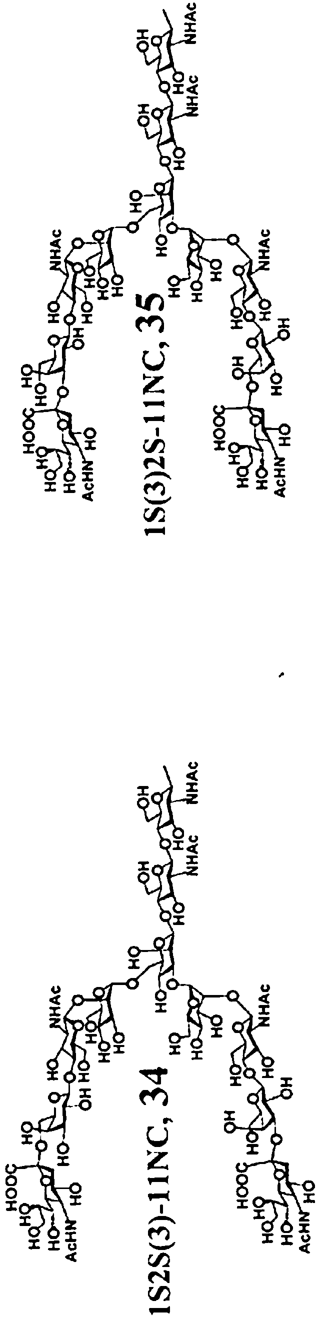

In one embodiment of the present disclosure, the solubilizing group according to the present disclosure has the following structure.

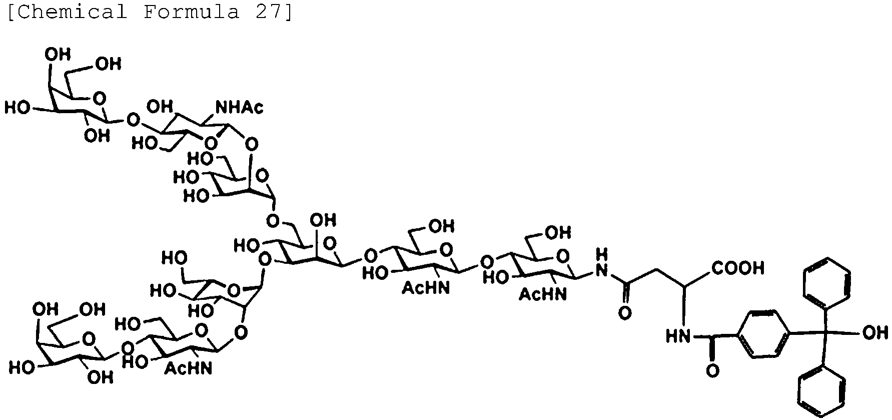

The solubilizing group according to the present disclosure can be produced according to a method well-known to those skilled in the art. For example, it can be produced by allowing dehydration condensation between the reactive group (such as amino group) of a glycosylated amino acid (such as glycosylated asparagine and glycosylated cysteine) having a desired sugar chain and the reactive group (such as carboxy group) of the functional group moiety according to the present disclosure. In an exemplary embodiment, e.g., by activating 4-(diphenyl hydroxymethyl)benzoic acid, and reacting this with a glycosylated amino acid such as glycosylated asparagine, a solubilizing group comprising triphenylmethanol as the functional group moiety can be obtained. The reaction time will vary depending on the reagents or solvents employed, and can be 1 minute - 48 hours, preferably 10 minutes - 24 hours, for example 6 hours. Moreover, the reaction temperature will vary depending on the reagents or solvents employed and can be -78°C - 300°C, preferably -78°C - 150°C, for example room temperature.

Reagents that can activate the carboxy group of the functional group moiety can include, but is not limited to, carbodiimide-based condensing agents such as 1-[bis(dimethylamino)methylene]-1H-1,2,3-triazolo[4,5-b]pyridinium 3-oxide hexafluorophosphate (HATU) and 1-ethyl-3-(3-dimethylaminopropyl)carbodiimide hydrochloride (WSCD), triazine-based condensing agents such as 4-(4,6-dimethoxy-1,3,5-triazine-2-yl)-4-methyl morpholinium chloride-n-hydrate (DMT-MM), thionyl chloride, lower alkyl haloformates such as ethyl chloroformate, sulfuric acid, and the like. The above reagents may be employed alone, or may be employed in combination of multiple types.

Solvents that can be employed in producing the solubilizing group according to the present disclosure are not particularly limited, and can include, e.g., alcohols such as methanol, ethanol, tert-butyl alcohol, 2-methoxy ethanol, and 1,1,1,3,3,3-hexafluoro-isopropanol (HFIP); ethers such as diethyl ether, diphenyl ether, tetrahydrofuran, and 1,2-dimethoxyethane; aromatic hydrocarbons such as chlorobenzene, toluene, and xylene; saturated hydrocarbons such as cyclohexane and hexane; amides such as N,N-dimethylformamide (DMF) and N-methylpyrrolidone (NMP); halogenated hydrocarbons such as dichloromethane and carbon tetrachloride; acetonitrile, dimethyl sulfoxide, pyridine, formic acid, acetic acid, trifluoroacetic acid, hydrochloric acid, sulfuric acid, ethyl acetate, acetone, methylethyl ketone, guanidine, and aqueous urea solution mixed with acetonitrile, and the like. The above solvents may be employed alone, or may be employed in combination of multiple types.

In another aspect, the present disclosure relates to a protein or peptide comprising one or more of said solubilizing groups. The protein or peptide according to the present disclosure typically has increased solubility to water due to having said solubilizing group being bound thereto.

In the present disclosure, the site where the solubilizing group binds to the protein or peptide is not particularly limited, and it can typically bind to the sulfhydryl group, the carboxy group, or the amino group of the amino acid side chain constituting the protein or peptide. In one embodiment, the solubilizing group according to the present disclosure binds to the sulfhydryl group of the amino acid side chain constituting the protein or peptide.

Moreover, the number of solubilizing groups that the protein or peptide according to the present disclosure has per molecule can be appropriately varied according to the size and solubility of the protein or peptide, the extent of solubility intended, and the like. One solubilizing group per 20 - 30 residues of amino acids may be employed, although it is not particularly limited thereto.

In the present disclosure, the introduction of the solubilizing group according to the present disclosure to a protein or peptide can be performed according to a method well-known to those skilled in the art. For example, by allowing the formation of a disulfide bond between the sulfhydryl group of a cysteine of the protein or peptide to be subject to solubilization and the solubilizing group according to the present disclosure, the solubilizing group according to the present disclosure can be introduced to the protein or peptide. The said reaction can be performed in an appropriate solvent, and for example, solvents such as, but not limited to, 1,1,1,3,3,3-hexafluoro-isopropanol (HFIP), trifluoroethanol, and the like can be employed.

In the above reaction, a catalyst may be employed as necessary, and for example, but not limited to, trifluoroethanol and the like can be used.

In another aspect, the present disclosure relates to a method for producing a solubilized protein or peptide (also referred to herein as the "method for producing solubilized proteins etc. according to the present disclosure"). The method for producing solubilized proteins etc. according to the present disclosure comprises a step of binding the solubilizing group according to the present disclosure to the sulfhydryl group, the carboxy group, or the amino group of one or more amino acid side chains constituting any protein or peptide (also referred to herein as the "binding step").

In the method for producing solubilized proteins etc. according to the present disclosure, said binding step can be performed in an appropriate solvent, and for example, can be performed by mixing the target protein and the solubilizing group according to the present disclosure in a solvent such as, but not limited to, 1,1,1,3,3,3-hexafluoro-isopropanol (HFIP) and trifluoroethanol. A catalyst may be employed as necessary for the said reaction, and for example, but not limited to, trifluoroethanol and the like can be used.

The reaction time in said binding step will vary depending on the reagents or solvents employed, and can be 1 minute - 48 hours, preferably 10 minutes - 24 hours, for example 6 hours. Moreover, the reaction temperature will vary depending on the reagents or solvents employed, and can be -10°C - 50°C, preferably -10°C - 40°C, for example room temperature.

In the method for producing solubilized proteins etc. according to the present disclosure, the target for introducing the solubilizing group according to the present disclosure may not necessarily be a protein with poor water solubility. For example, a method for obtaining the solubilized protein of interest by introducing the solubilizing group according to the present disclosure to a soluble protein that was chemically or genetically prepared as a fusion protein with a water-soluble peptide, and subsequently removing said water-soluble peptide is also within the scope of the present disclosure.

In another aspect, the present disclosure relates to a method for producing the protein of interest employing a protein or peptide with poor water solubility (also referred to herein as the "method for producing the protein according to the present disclosure"). Specifically, the method for producing the protein according to the present disclosure comprises a step of binding the solubilizing group according to the present disclosure to the sulfhydryl group, the carboxyl group, or the amino group of one or more amino acid side chains constituting proteins or peptides with poor water solubility to allow solubilization (also referred to herein as the "solubilization step"), and a step of linking said solubilized protein or peptide with another soluble protein or peptide, or solubilized protein or peptide, to obtain the protein of interest(also referred to herein as the "linking step").

The protein or peptide with poor water solubility to be solubilized in the solubilization step is a protein or peptide with poor water solubility that constitutes a portion of the protein of interest. Moreover, the protein or peptide with poor water solubility to be solubilized in the solubilization step may be one type or may be multiple types.

Said solubilization step can be performed in an appropriate solvent, and for example, can be performed by mixing the target protein and the solubilizing group according to the present disclosure in a solvent such as, but not limited to, 1,1,1,3,3,3-hexafluoro-isopropanol (HFIP) and trifluoroethanol. A catalyst may be employed as necessary for said reaction, and for example, but not limited to, trifluoroethanol and the like can be used.

The reaction time in said solubilization step will vary depending on the reagents or solvents employed, and can be 1 minute - 48 hours, preferably 10 minutes - 24 hours, for example 6 hours. Moreover, the reaction temperature will vary depending on the reagents or solvents employed, and can be -10°C - 50°C, for example room temperature.

The linking step is a method for linking the protein or peptide that was solubilized in the solubilization step with an intrinsically soluble protein or peptide (also referred to herein as the "soluble protein or peptide") or other proteins or peptides with poor water solubility that were solubilized by the method according to the present disclosure or a method that is different from the method according to the present disclosure. Solubilization by a method that is different from the method according to the present disclosure includes, but is not limited to, for example, chemical or genetic preparation as a fusion protein with a water-soluble peptide.

The linking of the solubilized protein or peptide/the soluble protein or peptide can be performed with any ligation method well-known to those skilled in the art. In one embodiment of the present disclosure, the linking step is carried out with Native Chemical Ligation (NCL method). The NCL method is a chemical selection reaction between a first peptide made to have an α carboxythioester moiety at the C-terminal and a second peptide having a cysteine residue at the N-terminal (such as International Publication No. 96/34878), and the reaction condition thereof, as well as the method for preparing the first and second peptides are also well-known to those skilled in the art (such as International Publication No. 2013/047372). Alternatively, the linking of the solubilized protein or peptide/the soluble protein or peptide may also be performed via a peptide linker and the like.

In one embodiment of the present disclosure, the method for producing the protein according to the present disclosure can further comprise detaching and collecting the solubilizing group according to the present disclosure after obtaining the protein of interest. In other words, depending on the constitution of the protein of interest, by linking with the water-soluble peptide or protein, solubility may be maintained even if the solubilizing group according to the present disclosure no longer exists. In such a case, it would be extremely beneficial if the solubilizing group could be detached and collected from the protein of interest, and the difficult-to-obtain and expensive sugar chain-containing solubilizing group could be used repeatedly.

Specifically, in one embodiment, the method for producing the protein according to the present disclosure further comprises a step of detaching said solubilizing group from said protein of interest in the presence of an acid, and a step of collecting said solubilizing group.

In the method for producing the protein according to the present disclosure, "in the presence of an acid" refers to the acidic condition generally employed in the deprotection of a protecting group. Acids that can be used in the method for producing the protein according to the present disclosure can include, but is not limited to, e.g., inorganic acids such as hydrochloric acid and sulfuric acid, Lewis acids such as boron trifluoride diethylether (BF3•OEt2), dimethyl(methylthio)sulfonium trifluoromethanesulfonate (DMTST), trimethylsilyl trifluoromethanesulfonate, triethylsilyl trifluoromethanesulfonate, tripropylsilyl trifluoromethanesulfonate, dimethylethylsilyl trifluoromethanesulfonate, tribenzylsilyl trifluoromethanesulfonate, trinaphthylsilyl trifluoromethanesulfonate, or tribenzylmethylsilyl trifluoromethanesulfonate, silver trifluoromethanesulfonate, cyclopentadienyl hafnium chloride, cyclopentadienyl zirconium chloride, and tin chloride, organic acids such as formic acid, acetic acid, trifluoroacetic acid, trifluoroacetic anhydride, trifluoromethanesulfonic acid, and tetrafluoromethanesulfonic acid, and the like. These acids can be used in a combination of one or two or more types. Moreover, those skilled in the art can appropriately set the appropriate amount of the amount of acids used, and it is preferred to be employed in excess amounts relative to the solubilized protein.

In the method for producing the protein according to the present disclosure, the collection of the detached solubilizing group can be performed with a method well-known to those skilled in the art. For example, the collection of the solubilizing group is not particularly limited as long as it is an approach that can separate materials based on molecular weight, charge, and other chemical natures, but it is preferred to employ liquid column chromatography.

Note that the terms used herein are to be employed to describe particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meaning as that broadly recognized by those skilled in the art of the technology to which the present disclosure belongs. The terms used herein, unless explicitly defined otherwise, should be construed as having meanings consistent with the meanings herein and in related technical fields, and shall not be construed as having idealized or excessively formal meanings.

Terms such as first and second may be employed to express various elements, but it should be recognized that these elements are not to be limited by these terms. These terms are employed solely for the purpose of discriminating one element from another, and for example, it is possible to describe a first element as a second element, and similarly, to describe a second element as a first element without departing from the scope of the present disclosure.

The present disclosure will now be more specifically described below by way of Examples. However, the present disclosure can be embodied by various forms, and it shall not be construed as being limited to the Examples described herein.

### Examples

### Example 1: Preparation of sugar chain solubilizing group

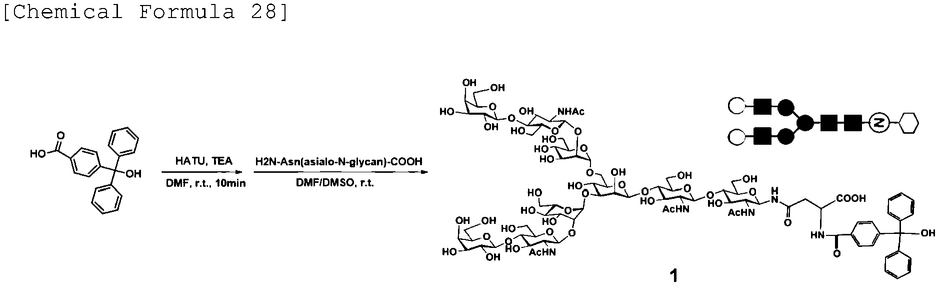

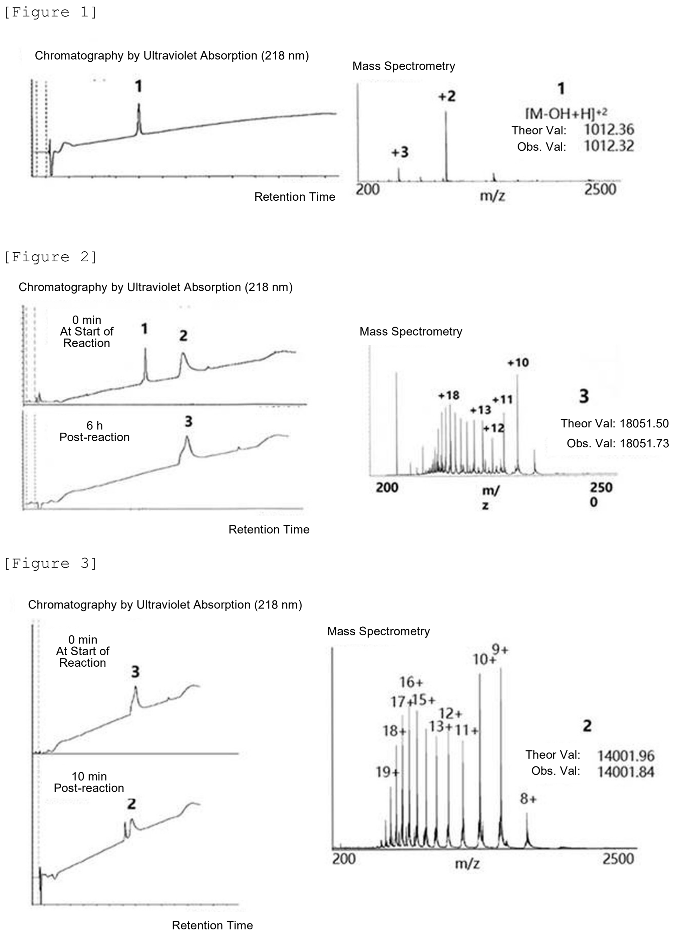

4-(diphenyl hydroxymethyl)benzoic acid (4.5 mg, 15 µmol, 6 equivalents; Sigma Aldrich^{™}) and 1-[bis(dimethylamino)methylene]-1H-1,2,3-triazolo[4,5-b]pyridinium 3-oxide hexafluorophosphate (HATU) (3.8 mg, 10 µmol, 4 equivalents; Novabiochem^{™}) were dissolved in dry DMF (150 µL; Kanto Chemical Co.,Inc.), and then triethylamine (1.4 µL, 10 µmol, 4 equivalents; Nacalai Tesque^{™}) was added. This mixture was stirred at room temperature for 10 minutes to activate 4-(diphenyl hydroxymethyl)benzoic acid. To this was added H₂N-Asn (asialo-N-glycan)-COOH (4.4 mg, 2.5 µmol, 1 equivalent) dissolved in dry DMF:DMSO (7:3, 250 µL), and this was reacted at room temperature. The reaction status was tracked with LC/MS, and completion was confirmed. After completion of the reaction, chilled diethyl ether was added to this solution, the product was precipitated, and the residue target material was isolated by centrifugation. The solid obtained was purified with reverse phase HPLC (Proteonavi column; gradient ratio A:B, 90/10 - 10/90, 60 minutes, 2.5 ml/min, solvent A: 0.1% TFA (in H₂O), solvent B: 0.1% TFA (in 90/10 acetonitrile/H₂O), and the target material 1 was obtained as colorless solid (3.5 mg, 75% yield) (Figure 1). ESI-MS [M-OH+H]+2 calculated 1012.36 found 1012.32

### Example 2: Sugar chain protection employing model peptide

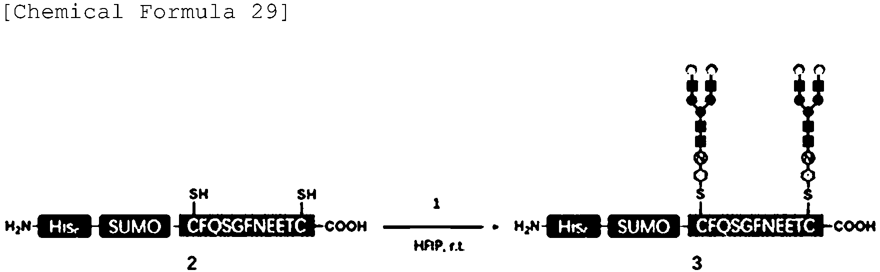

Compound 1 (1.4 mg, 0.686 µmol, 8 equivalents relative to the peptide) was dissolved in 1,1,1,3,3,3-hexafluoro-isopropanol (HFIP, 180 µL; Tokyo Chemical Industry Co.,Ltd.). To this yellow solution, peptide 2 (1.2 mg, 0.09 µmol) prepared with E. coli expression method was added to start the reaction. The reaction status was tracked with LC/MS, and completion was confirmed. After completion of the reaction, chilled diethyl ether was added to this solution, the product was precipitated, and after isolation of the residue target material by centrifugation, this was purified by reverse phase HPLC (Proteonavi column; gradient ratio A:B, 90/10 - 30/70, 60 minutes, 2.5 ml/min, solvent A: 0.1% TFA (in H₂O), solvent B: 0.1% TFA (in 90/10 acetonitrile/H₂O), and the target material 3 was obtained as colorless solid (1.0 mg, 80% yield) (Figure 2). ESI-MS [M+1H]+1 calculated 18051.50 found 18051.73

### Deprotection of sugar chain solubilizing group - 1

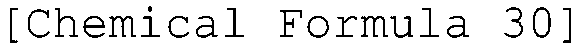

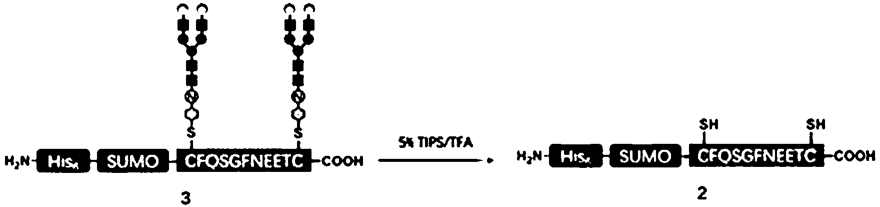

Compound 3 (1 mg, 0.56 µmol) was dissolved in 5% tetraisopropyl silane (TIPS; Tokyo Chemical Industry Co.,Ltd.)/TFA (500 µL; FUJIFILM Wako Pure Chemical Corporation), and this was reacted at room temperature for 10 minutes. After completion of the reaction, TFA was evaporated, chilled diethyl ether was added to the residual TFA solution, the product was precipitated, and after isolation of the residue target material by centrifugation, this was purified by reverse phase HPLC (Proteonavi column; gradient ratio A:B, 90/10 - 30/70, 60 minutes, 2.5 ml/min, solvent A: 0.1% TFA (in H₂O), solvent B: 0.1% TFA (in 90/10 acetonitrile/H₂O), and the target material 2 was obtained as colorless solid (0.7 mg, 90% yield) (Figure 3).

### Removal of SUMO tag protein by enzyme

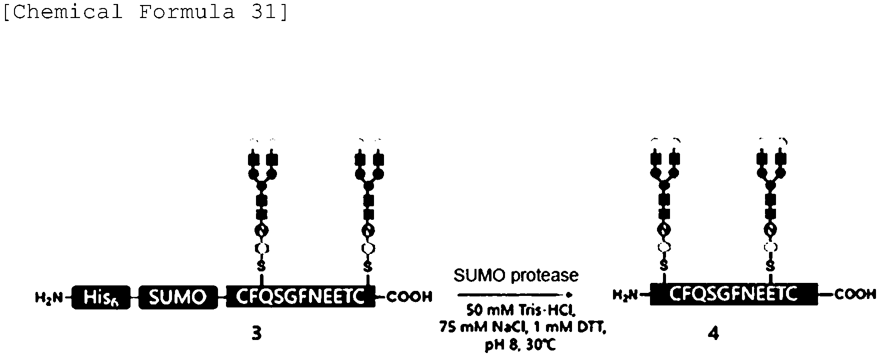

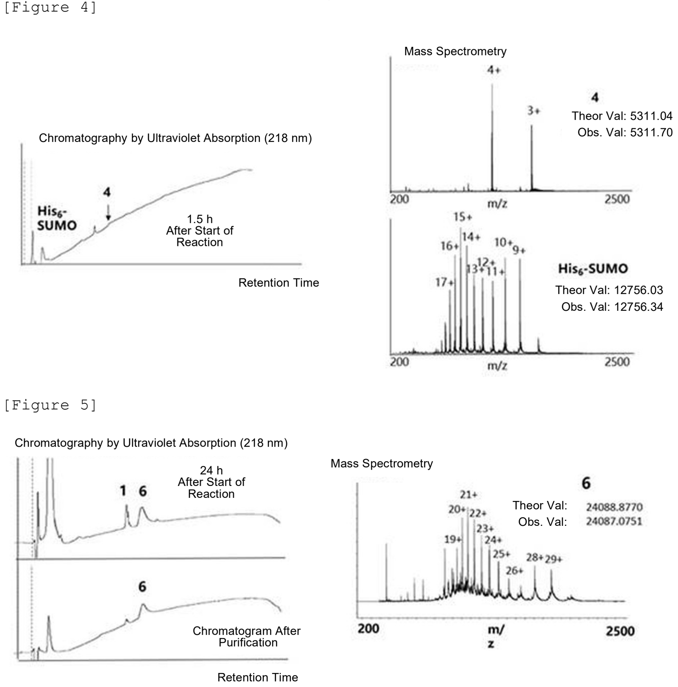

Removal of SUMO tag protein for solubilizing a hydrophobic peptide and HisTag for affinity purification was performed with SUMO protease. Compound 3 (1 mg, 0.56 µmol) was dissolved in tris buffer solution (50 mM Tris-HCl (FUJIFILM Wako Pure Chemical Corporation), 75 mM NaCl (FUJIFILM Wako Pure Chemical Corporation), and 1 mM dithiothreitol (DTT, FUJIFILM Wako Pure Chemical Corporation), pH 8.0), SUMO protease was added at an appropriate amount to allow the reaction to proceed, and this was tracked with LC/MS until completion of the reaction at 30°C. After completion of the reaction, the solution was directly purified with reverse phase HPLC (Proteonavi column; gradient ratio A:B, 90/10 - 30/70, 60 minutes, 2.5 ml/min, solvent A: 0.1% TFA (in H₂O), solvent B: 0.1% TFA (in 90/10 acetonitrile/H₂O), and the target material 2 was obtained as colorless solid (0.3 mg, 90% yield) (Figure 4). ESI-MS [M+H]+1 calculated 5311.04, found 5311.70

### Example 3: Sugar chain protection of interleukin-6 (IL-6) N-terminal fragment (1-141) that utilizes sugar chain solubilizing group

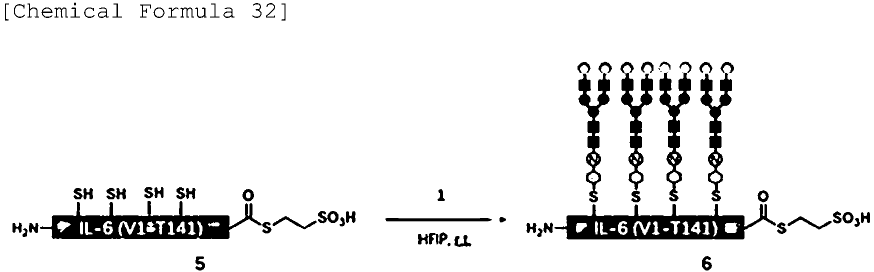

Compound 1 (1 mg, 0.49 µmol, 16 equivalents relative to the peptide) was dissolved in 1,1,1,3,3,3-hexafluoro-isopropanol (HFIP, 60 µL). To this yellow solution, peptide 5 (0.5 mg, 0.03 µmol) prepared with E. coli expression method was added and dissolved to start the reaction. The reaction status was tracked with LC/MS, and completion was confirmed. After completion of the reaction, chilled diethyl ether was added to this solution, the product was precipitated, and after isolation of the residue target material by centrifugation, this was purified by reverse phase HPLC (Proteonavi column; gradient ratio A:B, 70/30 - 20/80, 30 minutes, 1.0 ml/min, solvent A: 0.1% TFA (in H₂O), solvent B: 0.1% TFA (in 90/10 acetonitrile/H₂O), and the target material 6 was obtained as colorless solid (0.4 mg, 80% yield) (Figure 5). ESI-MS[M+H]+1 calculated 24088.8770, found 24087.0751

### Deprotection of sugar chain solubilizing group - 2

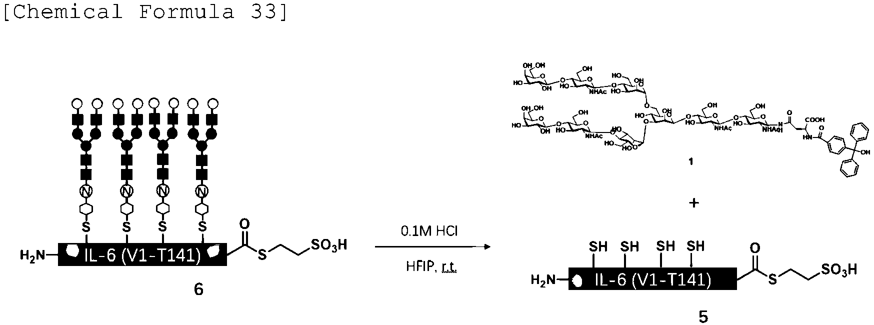

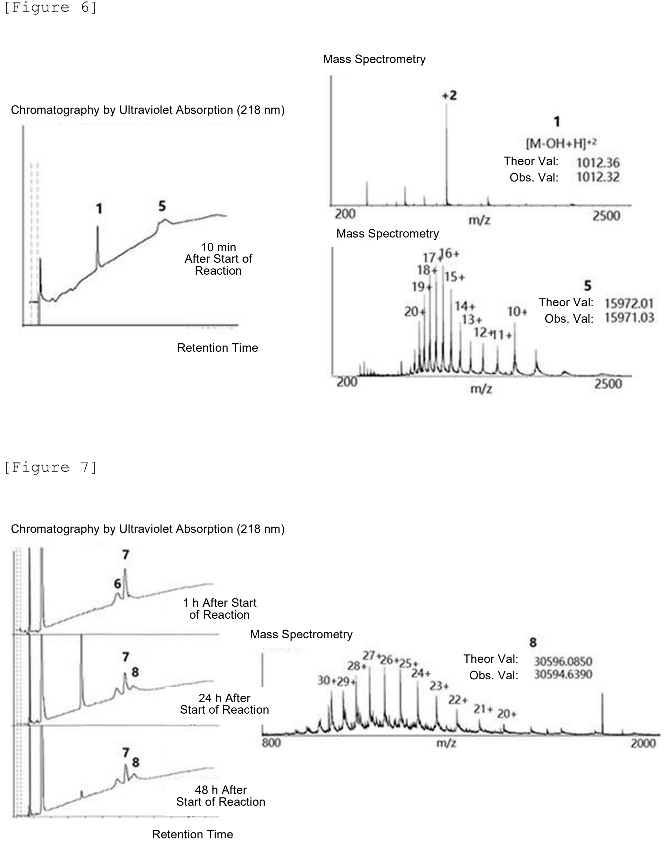

Compound 6 (0.4 mg, 0.02 µmol, 1 equivalent) was dissolved in 0.1 M HCl/HFIP solution (25 µL), and the sugar chain solubilizing group was removed. The reaction was tracked with LC/MS, and after completion of the reaction, tris buffer solution (6 M Gn-HCl (FUJIFILM Wako Pure Chemical Corporation) and 50 mM Tris-HCl, pH 8.0, 500 µL) was added, this was directly purified with reverse phase HPLC (Proteonavi column; gradient ratio A:B, 90/10 - 10/90, 60 minutes, 2.5 ml/min, solvent A: 0.1% TFA (in H₂O), solvent B: 0.1% TFA (in 90/10 acetonitrile/H₂O), and target materials 5 and 1 were obtained (Figure 6). The collected sugar chain solubilizing group was confirmed to have the structure of 1.

### Example 4: Synthesis of hydrophobic glycoprotein IL6 utilizing native chemical ligation and sugar chain solubilizing group

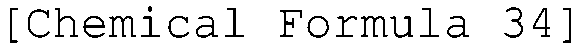

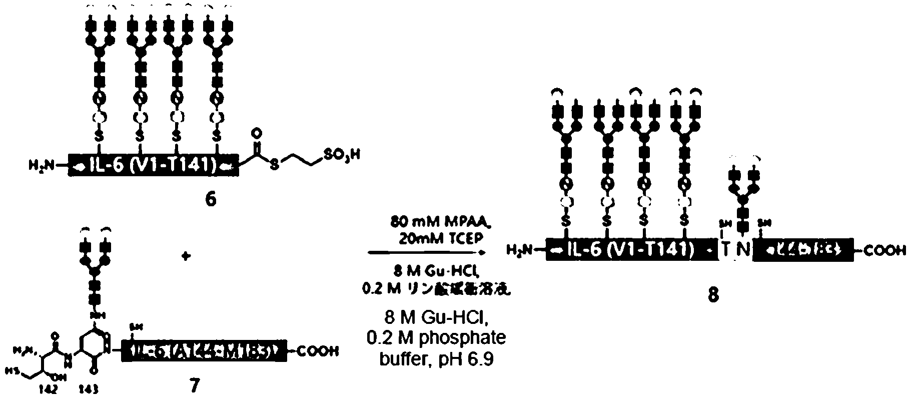

Compound 6 (1.1 mg, 0.05 µmol) was dissolved in 200 mM phosphate buffer, and each mixture was adjusted to have the following concentrations (compound 6: 2.0 mM, 8 M Gu-HCl, 80 mM 4-mercaptophenylacetic acid: MPAA, FUJIFILM Wako Pure Chemical Corporation, 20 mM tris (2-carboxyethyl)phosphine: TCEP, Tokyo Chemical Industry Co.,Ltd., pH 6.9). To this solution was dissolved compound 7 (0.5 mg, 0.10 µmol) having sugar chains bound thereto with E. coli expression method and liquid phase method, and peptide linking reaction was performed. The reaction was performed at room temperature for 2 days. Progress was checked by LC/MS by adding TCEP to the reaction solution. After completion of the reaction, this was purified with gel filtration column of HPLC (column: TSK gel Super SW3000, 4.6 mm x 30 cm, 4 µm). 0.1 M phosphoric acid solution comprising 6 M Gu-HCl (pH 6.7, 0.2 ml/min, 35 minutes) was used as the mobile phase to obtain 8 (Figure 7). ESI-MS [M+H]+1 calculated 30596.0850,found 30594.6350.

The product obtained was directly employed for the next desulfurization experiment.

### Desulfurization reaction of thiol in the presence of sugar chain solubilizing group

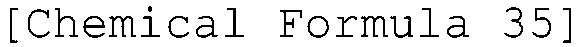

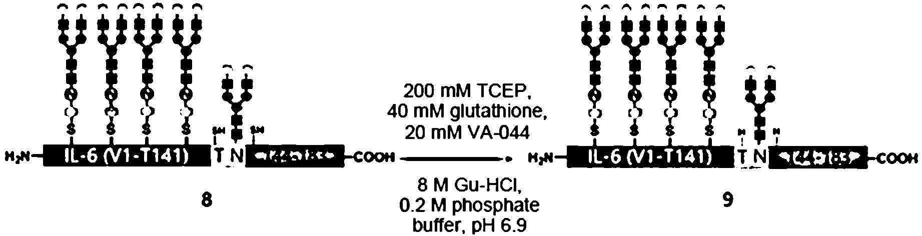

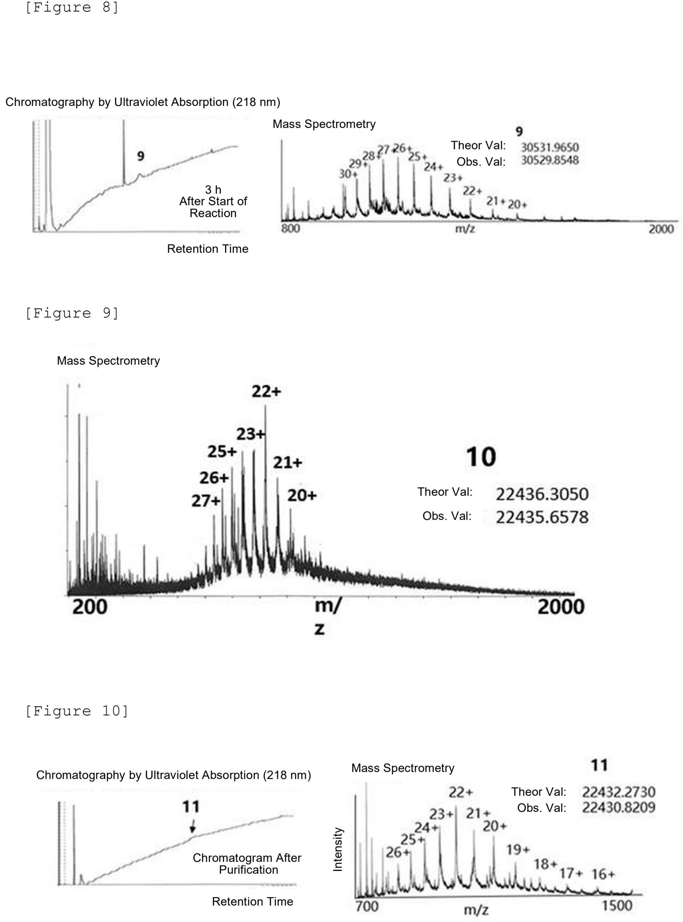

Compound 8 obtained with purification by gel filtration was dissolved in buffer solution (8 M Gu-HCl, 0.2 M phosphate, 200 mM TCEP, 40 mM glutathione (Sigma Aldrich^{™}), and 20 mM VA-044 (FUJIFILM Wako Pure Chemical Corporation)), and this was left at room temperature to allow desulfurization of thiol. The reaction was tracked with LC/MS, and after completion of the reaction, this was directly purified with reverse phase HPLC (Proteonavi column; gradient ratio A:B, 70/30 - 10/90, 60 minutes, 1.0 ml/min, solvent A: 0.1% TFA (H₂O), solvent B: 0.1% TFA (in 90/10 acetonitrile/H₂O), and the target material 9 was obtained as colorless solid (0.9 mg, 60% yield in two steps) (Figure 8). ESI-MS [M+H]+1 calculated 30531.9650, found 30529.8548

### Deprotection of sugar chain solubilizing group employing IL-6

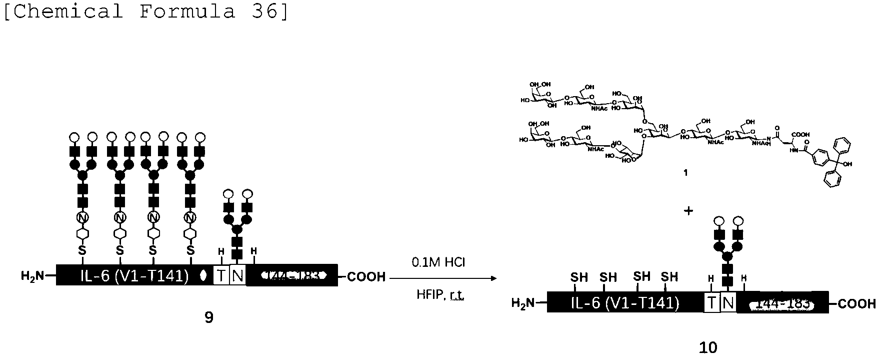

Compound 9 (0.9 mg, 0.03 µmol) was dissolved in HFIP, and HCl solution was added to have 100 mM. This solution was left at room temperature, LC/MS confirmed that the sugar chain solubilizing group was removed (Figure 9), and protein folding operation was directly carried out.

### Removal of sugar chain solubilizing group and folding of IL-6

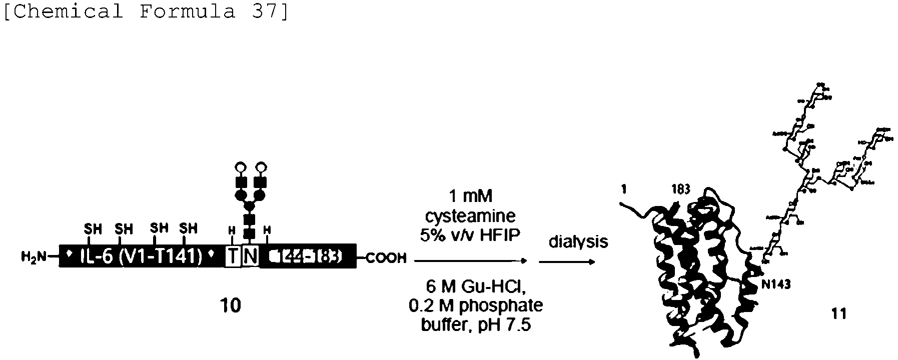

To HFIP solution comprising compound 10, 50 mM tris buffer solution comprising 6 M Gu-HCl and 1 mM cysteamine (FUJIFILM Wako Pure Chemical Corporation) (final pH at 7.3 - 7.5) was added, and this was left at room temperature. The folding reaction was tracked with LC/MS, and after folding, dialysis was performed with 50 mM tris buffer solution (pH = 8), and folded IL-6 11 was obtained (Figure 10). ESI-MS [M+H]+1 calculated 22432.2730, found 22430.8209

## Claims

1. A hydrophilic solubilizing group for proteins or peptides, comprising a sugar chain moiety; and a functional group moiety that is bound to the amino or carboxy group of said sugar chain, and that binds to the amino acid side chain and can be detached from said protein or amino acid side chain in the presence of an acid.

2. The solubilizing group according to Claim 1, comprising a sugar chain moiety; and a triphenylmethanol moiety bound to the amino or carboxy group of said sugar chain.

3. The solubilizing group according to Claim 2, wherein said triphenylmethanol moiety is bound to the carboxy group of said sugar chain.

4. The solubilizing group according to Claim 1, wherein said sugar chain is **characterized in that** it consists of 4 or more sugars.

5. The solubilizing group according to Claim 1, wherein said sugar chain is **characterized in that** it consists of 7 or more sugars.

6. The solubilizing group according to Claim 1, wherein said sugar chain is **characterized in that** it consists of 9 or more sugars.

7. The solubilizing group according to Claim 1, wherein said sugar chain is **characterized in that** it is a complex type bianntennary sugar chain, a complex type triantennary sugar chain, or a complex type tetraantennary sugar chain.

8. The solubilizing group according to Claim 1, wherein said sugar chain is **characterized in that** it is a complex type bianntennary sugar chain.

9. The solubilizing group according to Claim 8, wherein said complex type bianntennary sugar chain is **characterized in that** it is a sugar chain selected from the group consisting of a disialo sugar chain, a monosialo sugar chain, an asialo sugar chain, a diGlcNAc sugar chain, and a dimannose sugar chain.

10. The solubilizing group according to Claim 1, wherein said sugar chain is **characterized in that** it is a sugar chain represented by the following formula: 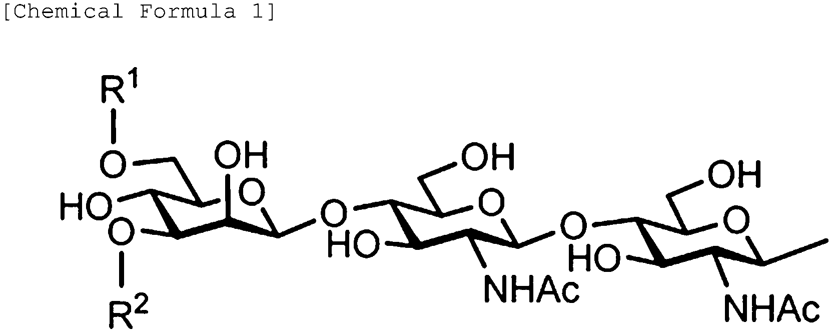 [wherein R¹ and R² are the same or different, to indicate 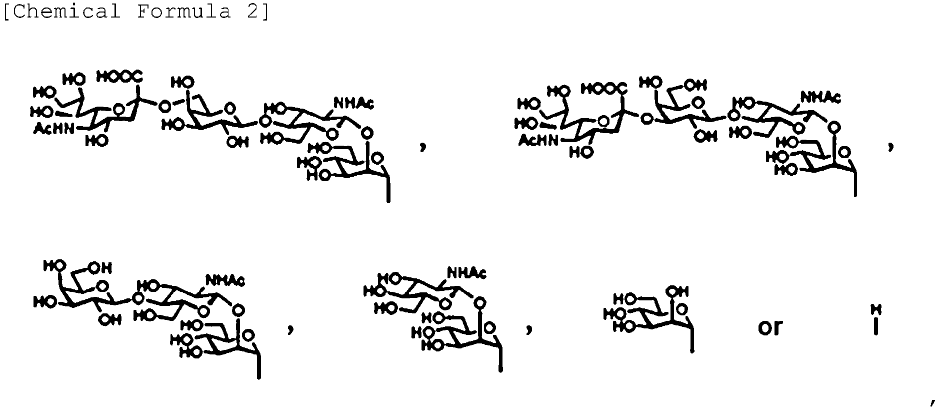 and Ac indicates an acetyl group].

11. The solubilizing group according to Claim 1, having the following structure.

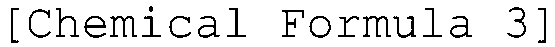

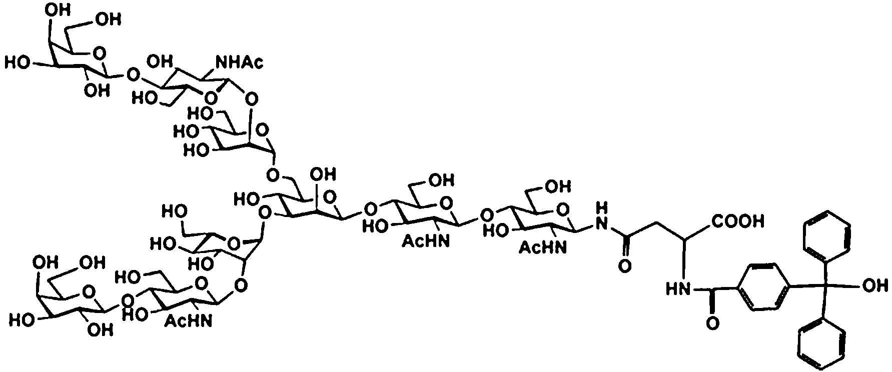

12. A peptide or protein having one or more of the solubilizing groups according to Claim 1.

13. The peptide or protein according to Claim 12, which has poor water solubility.

14. The peptide or protein according to Claim 12, selected from the group consisting of interleukin and transmembrane protein, and a fragment thereof.

15. The peptide or protein according to Claim 14, which is rhodopsin or IL-6, or a fragment thereof.

16. A method for producing a solubilized protein or peptide, comprising a step of binding the solubilizing group according to Claim 1 to the sulfhydryl group, the carboxy group, or the amino group of one or more amino acid side chains constituting any protein or peptide.

17. A method for producing the protein or peptide of interest employing proteins or peptides with poor water solubility, comprising:
a step of binding the solubilizing group according to Claim 1 to the sulfhydryl group, the carboxy group, or the amino group of one or more amino acid side chains constituting said proteins or peptides with poor water solubility to allow solubilization, and
a step of linking said solubilized protein or peptide with another soluble protein or peptide, or solubilized protein or peptide, to obtain the protein of interest.

18. The method according to claim 17, further comprising:
a step of detaching said solubilizing group from said protein of interest in the presence of an acid, and
a step of collecting said solubilizing group.
